Europäisches Patentamt

European Patent Office

Office européen des brevets

(19)

(11) **EP 1 199 159 A1**

(12) **EUROPEAN PATENT APPLICATION**

(43) Date of publication:
**24.04.2002 Bulletin 2002/17**

(51) Int Cl.7: **B32B 27/08**, B32B 27/12,
D06N 7/00, A61L 15/42,
A61L 15/22

(21) Application number: **00122216.5**

(22) Date of filing: **16.10.2000**

(84) Designated Contracting States:
**AT BE CH CY DE DK ES FI FR GB GR IE IT LI LU MC NL PT SE**
Designated Extension States:
**AL LT LV MK RO SI**

(71) Applicant: **The Procter & Gamble Company**
**Cincinnati, Ohio 45202 (US)**

(72) Inventors:
• **Toro, Carlo**
**65012 Cepagatti (Pescara) (IT)**

• **Carlucci, Giovanni**
**66100 Chieti (IT)**
• **Elliott, Russell Phillip**
**Egham, Surrey TW20 9RJ (GB)**
• **Duke, Roland Philip**
**Wokingham, Berkshire RG41 3NB (GB)**

(74) Representative: **Kremer, Véronique et al**
**Procter & Gamble Service GmbH**
**65824 Schwalbach am Taunus (DE)**

(54) **A non-slip moisture vapour permeable composite textile structure and absorbent articles comprising the same**

(57)    The present invention relates to non slip, liquid impermeable moisture vapour permeable composite structures comprising a hydrophilic thermoplastic film which is coated onto a support layer, the support layer having a coefficient of friction greater than 1. The composite structures of the present invention can be used in a variety of applications wherein moisture vapour permeability and anti-slipping properties are desirable, such as in absorbent articles for example breast pads, perspiration pads, diapers, sanitary napkins, panty liners and incontinence products, and also as protective bedding covers, protective clothing and the like.

Fig. 1

**Description**

Field of the Invention

The present invention relates to non-slip, liquid impermeable, moisture vapour permeable composite structures comprising a hydrophilic thermoplastic film which is typically thinly coated onto a support layer. The composite structures of the present invention can find a variety of applications, wherein anti-slipping properties and moisture vapour permeability are desirable for example within absorbent articles such as diapers, sanitary napkins, panty liners, breast pads and incontinence products, protective bedding covers, protective clothing and the like.

Background of the Invention

Liquid impermeable, moisture vapour permeable materials are well known in the art. Various absorbent articles like sanitary napkins or pantiliners to be worn in close proximity to the uro-genital area of a woman are typically made of such materials on their garment/undergarment facing side to prevent undesirable soil through occurrence while still being comfortable to wear. Usually such garment facing side is provided with attachment means, typically adhesive attachment means, that becomes available for use after removal of a paper release.

For some applications, however, non-slip properties in liquid impermeable, moisture vapour permeable material/layer, are appropriate. Such applications include diapers, protective bedding covers like mattress covers, protective clothing, perspiration pads and especially breast pads to be used by nursing mothers or any woman in need thereof due for instance of transpiration problems.

For example it is desirable to establish an increased friction between the surface of a breast pad facing the garment (bra) and the garment (bra), as the breast pad might typically be subjected to certain tension or pressure (for instance when the mother is carrying her baby in front of her breasts and is faced with her baby's movements), in order to minimise possible displacements of the breast pad during its use. Increased friction prevents or at least reduces relative movements between the breast pad and the bra, therefore helping in keeping the breast pad in its right position provided initially, e.g., by the woman when applying the breast pad against its breast in the bra.

Thus, while there are various laminate and composite materials available for use in applications where liquid impermeability and moisture vapour permeability are required, there nevertheless remains a need for such materials having non-slip properties for various applications.

Indeed it is an object of the present invention to provide a non-slip, laminate or composite material, particularly suitable for use as a garment facing surface (also called backsheet) of an absorbent article like a breast pad, while delivering effective liquid impermeability and moisture vapour permeability. More particularly it is an object of the present invention to provide such a non-slip laminate or composite material which can be used in an absorbent article like a breast pad, while delivering effective protection with reduced wet through /soiling occurrence, thereby improving overall comfort in use and discretion. It is another object of the present invention to provide such a non-slip laminate or composite material which can be used in an absorbent article like a breast pad, without impairing on the flexibility, and hence overall conformability of such an article. It is a further object of the present invention to provide such a non-slip laminate or composite material which can be used in an absorbent article like a breast pad, without impairing on the tensile strength of such an article during use, and hence their integrity under wet conditions.

It is a further object of the present invention to provide such a non slip material and hence absorbent articles comprising such a material in an easy and cost effective manner.

This has been achieved by providing a non-slip, moisture vapour permeable, liquid impervious (also called herein impermeable) composite structure comprising a support layer having a first and a second opposing surfaces, wherein the first surface is facing an hydrophilic thermoplastic film and the second surface has a coefficient of friction greater than 1. Typically such composite structure might be used in absorbent articles, for example breast pads, as the so called breathable backsheet, the second surface of the support layer being the surface facing the wearer's bra.

Advantageously the composite structure according to the present invention when used as an outer layer for absorbent articles or other paper articles like bedding covers, gloves and the like provides outstanding liquid protection by reducing leakage and soiling through and by staying in place during use conditions. Indeed due to its stay in place properties when used as an outer layer of an article like a breast pad, namely as the layer facing the garment (e.g. bra), its usage avoids that wet skin comes in direct contact with the bra. By providing outstanding liquid protection with stay in place properties improved overall comfort and discretion in use is delivered.

Advantageously the present invention provides non-slip, liquid impermeable, moisture vapour permeable composite structures with excellent moisture vapour permeability while taking the advantage of ease of manufacturing. Indeed readily processability of thin non-slip film coating are achieved.

Background art of the invention

Moisture vapour permeable composite structures and their utilisation thereof in absorbent structures as well as low viscosity thermoplastic compositions for making such moisture vapour permeable structures are known from EP-A-963837, EP-A-963760 and EP-A-964026. The disclosed thermoplastic compositions are readily processable so as to provide a coating having the desired thickness onto a substrate, so avoiding the need of complex traditional extrusion apparatuses. This is achieved by modifying the viscosity of the thermoplastic polymers by means of the inclusion in the composition of a suitable plasticiser or blend of plasticisers that lowers such viscosity. This allows to use with these preferred compositions typical process conditions known in the art for the direct coating of low viscosity hot melt compositions onto a substrate in order to form a moisture vapour permeable, liquid impervious film or layer. However all these references fail to disclose non-slip, moisture vapour permeable, liquid impervious composite structures, let alone absorbent articles using such composite structures, and hence absorbent articles like breast pads having anti-slip properties while providing excellent moisture vapour permeability properties.

Brief description of the drawings

The invention is further described with reference to the accompanying drawings:

Figure 1 is an enlarged perspective view, partly in cross section, of a composite structure of the present invention showing layer of thermoplastic film applied onto to the inner layer of support layer, the outer layer of the support layer comprising a discontinuous pattern of anti-slip material.

Figure 2 is a perspective view similar to Figure 1 showing an alternate form of surface treatment of the support layer in which the surface has a grid pattern of anti-slip material.

Summary of the invention

The present invention encompasses a non-slip, moisture vapour permeable, liquid impervious composite structure comprising a hydrophilic thermoplastic film and a support layer, the support layer having a first and a second opposing surfaces, wherein the first surface of the support layer is facing the hydrophilic thermoplastic film and the second surface has a coefficient of friction greater than 1.
The present invention also encompasses an absorbent article, especially breast pad, comprising such a non-slip, moisture vapour permeable liquid impervious composite structure.

Detailed description of the invention

By 'hydrophilic thermoplastic film' it is meant herein a continuous film that do not allow the flow of moisture vapour through open pores or apertures in the material, but do transfer substantial amounts of moisture vapour through the film by absorbing water on one side of the film where the moisture vapour concentration is higher, and desorbing or evaporating it on the opposite side of the film where the moisture vapour concentration is lower.
According to the present invention the composite structures comprise a hydrophilic thermoplastic film made from a moisture vapour permeable thermoplastic composition.
Suitable thermoplastic compositions for use herein are thermoplastic compositions known to those skilled in the art for making hydrophilic continuous moisture vapour permeable, liquid impermeable layers or films ("monolithic films") having the characteristics of moisture vapour permeability and liquid imperviousness.
Typically the thermoplastic film of the composite structures according to the present invention is made from a moisture vapour permeable thermoplastic composition which comprises as an essential component from 5% to 100% by weight of said film of a polymer or mixture of polymers. More preferably the thermoplastic composition comprises from 10% to 80% and most preferably from 25% to 70% by weight of said polymer.
The thermoplastic compositions for use herein typically comprise thermoplastic polymers such as polyurethanes, poly-ether-amides block copolymers, polyethylene-acrylic acid copolymers, polyethylene oxide and its copolymers, ethylene acrylic esters copolymers, poly lactide and copolymers, polyamides, polyester block copolymers, sulfonated polyesters, poly-ether-ester block copolymers, poly-ether-ester-amide block copolymers, polyacrylates, polyacrylic acids and derivatives, ionomers, polyethylene-vinyl acetate with a vinyl acetate content of more than 28 weight %, polyvinyl alcohol and its copolymers, polyvinyl ethers and their copolymers, poly-2-ethyl-oxazoline and derivatives, polyvinyl pyrrolidone and its copolymers, thermoplastic cellulose derivatives, or mixtures thereof.
Particularly preferred thermoplastic polymers are thermoplastic poly-ether-amide block copolymers (e.g. Pebax™), thermoplastic poly-ether-ester-amide block copolymers, thermoplastic polyester block copolymers (e.g.

Hytrel™), thermoplastic polyurethanes (e.g. Estane™), or mixtures thereof.

Such thermoplastic polymers or mixture of polymers can be typically highly viscous in the melted state at the process conditions that are typical of the known processes of film or layer formation, e.g. an extrusion process involving a high power screw extruder. For example they may have a viscosity higher than 5000 poise at a temperature of 20°C above the DSC (Differential Scanning Calorimetry) melting point, which is the temperature identified as that corresponding to the DSC peak, or corresponding to the highest DSC peak in case of a mixture of polymers showing more than one peak, and at a frequency of 1 rad/sec.

The viscosity of the preferred thermoplastic polymers or mixture of polymers is adjusted by including in the thermoplastic composition a suitable plasticiser, or blend of plasticisers, that is compatible with the thermoplastic polymer(s) and that lowers the viscosity of the thermoplastic polymer or mixture of polymers in the melted state.

The thermoplastic compositions according to the present invention comprising the suitable plasticiser or blend of plasticisers have the following complex viscosities ($\eta^*$):

50 poise < $\eta^*$ < 4000 poise, preferably 100 poise < $\eta^*$ < 2000 poise, more preferably 100 poise < $\eta^*$ < 1000 poise, at a frequency of 1 rad/s at a temperature of 210°C or less and $\eta^*$ < 2000 pose, preferably $\eta^*$ < 1000 poise, more preferably $\eta^*$ < 500 poise, at a frequency of 1000 rad/s at a process temperature (T) of 210°C or less, wherein $\eta^*$ represents the complex viscosity of the thermoplastic polymeric composition. Preferably the temperature T is 200°C or less and more preferably 180°C or less and most preferably from 200°C to 50°C.

The thermoplastic compositions having the complex viscosity described allow for a film or layer to be coated onto a support layer/substrate using typical coating conditions and apparatuses known in the art for the coating of low viscosities hot melt compositions in a layer having a required thickness onto a support layer/substrate, while also keeping the advantageous characteristics of the preferred thermoplastic polymers in providing hydrophilic continuous moisture vapour permeable, liquid impermeable layers or films.

Thermoplastic compositions having such viscosities can also provide very thin films or layers. Typically the thermoplastic film used in the composite structures of the present invention have a thickness of less than 200 microns, preferably less than 130 microns, more preferably less than 80 microns, even more preferably from 70 to 0.5 microns and most preferably from 55 to 2 microns.

Advantageously the films used herein allow for films to be produced at greatly reduced thickness, whilst maintaining the mechanical properties of the film such that no failures concerning the uniformity of the film such as breakages or the presence of apertures arise.

Suitable plasticisers or blend of plasticisers for adjusting such viscosity can be selected from the group consisting of citric acid esters, tartaric acid esters, glycerol and its esters, adipates, sebacates, sorbitol, epoxidized vegetal oils, polymerised vegetal oils, polyols, phthalates, liquid polyesters, glycolates, p-toluene sulfonamide and derivatives, glycols and polyglycols, sorbitan esters, phosphates, monocarboxylic fatty acids ($C_8$-$C_{22}$) and their derivatives, and mixtures thereof. This allows to utilise with these preferred compositions typical process conditions known in the art for the direct coating of low viscosity hot melts onto a substrate in order to form a moisture vapour permeable, liquid impervious film or layer.

It has also been found that by further selecting the plasticiser or blend of plasticisers to be comprised in the thermoplastic composition used herein from the group of hydrophilic plasticisers consisting of acids, esters, amides, alcohols, polyalcohols, or mixtures thereof, the advantage of an enhanced moisture vapour permeability of the resulting layer or film formed from the thermoplastic composition is also achieved, when compared to a corresponding film or layer formed from a thermoplastic composition comprising the same thermoplastic polymer, but without such a plasticiser.

The preferred hydrophilic plasticiser or blend of hydrophilic plasticisers can of course also adjust the viscosity of the thermoplastic composition for use herein to the preferred values in order to make it processable by coating said thermoplastic composition onto a substrate in a layer or film having a desired thickness.

Suitable preferred hydrophilic plasticisers according to this preferred embodiment herein are citric acid esters, tartaric acid esters, glycerol and its esters, sorbitol, glycolates, and mixtures thereof.

Thus in addition to the thermoplastic polymeric component, the thermoplastic compositions also preferably further comprise a plasticiser or blend of plasticisers, preferably from 0% to 95%, more preferably from 20% to 90%, most preferably from 25% to 75% by weight of said thermoplastic composition.

The thermoplastic compositions from which the polymeric films of the composite structures of the present invention are made may in addition comprise additional optional components to further improve the processibility of the film and mechanical characteristics as well as other characteristics as its tackiness, its resistance to ageing by light and oxygen, its visual appearance etc.

Such optional components include tackifying resins or blends of tackifying resins having a softening point of 125°C or less. Preferred resins, which may be present by up to 50% by weight, may be selected from rosins and rosin esters, hydrocarbon resins, aliphatic resins, terpene and terpene-phenolic resins, aromatic resins, synthetic $C_5$ resins, mixtures of synthetic $C_5$-$C_9$ resins, and mixtures thereof.

Particularly suitable tackifier resins for use herein are those described in our European patent application No. 00116284.1, filed on 10 August 2000 with title: "Thermoplastic hydrophilic polymeric compositions with improved adhesive properties for moisture vapour permeable structures" (P&G Case CM2406F).

In a particular embodiment herein wherein it is desirable to provide increased friction properties of the thermoplastic film this can be achieved by suitably selecting the tackifier resin, or blend of tackifier resins, to be added to the thermoplastic composition. Indeed, it is possible to adjust the residual tackiness of said thermoplastic composition at room temperature. This in turn allows an adjustment of the friction that is established, in the use conditions, between the layer made of said thermoplastic composition comprised in the composite structure and any other layers adjacent said layer (typically the support layer and/or any other layer that might be adjacent said layer made of thermoplastic composition for example the core of an absorbent article when the composite structure is used as a backsheet of an absorbent article, the thermoplastic film having two opposing surfaces, one facing the directly the core of the absorbent article and one facing the support layer). In another embodiment of the present invention the thermoplastic film may face the wearer's garment (when used as the outer surface facing the wearer's garment) or a mattress surface (when used as the outer surface of a mattress cover). An increased friction provides in fact a better stability of the liquid impermeable, moisture vapour permeable thermoplastic film with respect to surface of adjacent layers, avoiding or at least reducing the relative movements.

By suitably selecting the tackifier resin or blend of tackifier resins for use herein it is also possible to adjust the residual tackiness at room temperature of the thermoplastic composition, and therefore of the layer formed from said thermoplastic composition, to the extent that it has the typical characteristics of a pressure sensitive adhesive. In other words, the friction between the layer of thermoplastic composition comprised in the composite structure of the present invention can be increased such that it can actually stick to the surface to which it is applied to, owing to the thermoplastic composition which can be formulated in order to behave like a pressure sensitive adhesive. Residual tackiness of the thermoplastic composition at room temperature can be suitably tailored since a smooth transition exists between increasing friction and actual adhesiveness.

Accordingly particularly preferred tackifier resins, or blend of tackifier resins to be selected herein have a softening point of 125°C or less, and are selected from the group consisting of rosin and rosin esters, terpene-phenolic resins, aromatic resins, and mixtures thereof.

Most preferably, the thermoplastic compositions for use herein comprise a blend of tackifier resins, preferably selected as described above, wherein moreover from 0% to 20%, preferably from 2% to 15%, by weight of said blend of tackifier resins comprises a resin or blend of resins having a softening point of less than 25°C, and from 80% to 100%, preferably from 85% to 98%, by weight of said blend of tackifier resins comprises a resin or blend of resins having a softening point of at least 70°C.

The presence in the blend of tackifier resins of a certain amount of one or more tackifier resins which are liquid at room temperature is preferred since said resin or resins, in addition to adjusting the residual tackiness of the layer formed from the resulting thermoplastic composition, also contribute to lower the viscosity of the composition itself at the process conditions, in combination with the selected suitable plasticiser or blend of plasticisers.

Said preferred blend of tackifier resins therefore provides a thermoplastic composition for forming the thermoplastic film in the composite structures of the present invention, which is easily processable. Moreover the preferred blend of tackifier resins allows to adjust the residual tackiness of the resulting thermoplastic film made from the thermoplastic composition, and therefore the friction properties towards the surface it is applied to as desired.

The thermoplastic compositions for making the thermoplastic film of the composite structures according to the present invention typically comprises from 0% to 60%, preferably from 2% to 60%, more preferably from 5% to 50%, and most preferably from 10% to 40%, by weight of said thermoplastic composition, of a tackifier resin or blend of tackifier resins.

Other optional components include anti-oxidants, anti-ultraviolets, pigments and mixtures thereof, which may be present within the thermoplastic composition at a level of up to 10% by weight of the composition.

A thermoplastic composition used herein can be manufactured with a process that will typically comprise the steps of providing the thermoplastic polymer or mixture of polymers, the optional plasticiser or blend of plasticisers, and the optional tackifier resin or blend of tackifier resins, heating the components and compounding them, e.g. with a known suitable mixer to form the thermoplastic composition in the molten state having the desired complex viscosity $\eta^*$.

According to the present invention a moisture vapour permeable, liquid impervious composite structure can be formed from the thermoplastic composition described herein by coating said thermoplastic composition onto a support layer/substrate. The films (also called layers) formed from the thermoplastic compositions of the present invention preferably have a moisture vapour transport rate of at least 100 g/m²·24h, preferably at least 300 g/m²·24h, most preferably at least 500 g/m²·24h with a thickness of the layer or film of at least 0.5 μm.

A process for making a layer or film from a thermoplastic composition as described herein typically comprises the steps of providing said composition, heating it to make it flowable, and coating said composition in the molten state onto a substrate in a layer having the desired thickness. While said substrate can be simply a formation substrate, onto which the thermoplastic composition is coated in order to form a film or layer of the desired thickness which is subsequently separated from said substrate and used as such, in a preferred embodiment of the present invention a moisture vapour permeable, water impervious composite is formed which comprises the thermoplastic composition and a support layer/substrate onto which said thermoplastic composition is coated, wherein the support layer/substrate is also moisture vapour permeable.

Such embodiment provides a moisture vapour permeable, liquid impervious composite structure wherein the contribution of the film formed from the thermoplastic composition described herein to the performance of the composite material resides only in the provision of a liquid barrier and hence could be advantageously provided as thinly as possible. The remaining performance physical criterion being preferably provided by the provided structure, that therefore preferably acts also as a support layer.

The substrate, or support layer may be any useful layer which is also moisture vapour permeable, preferably having a moisture vapour permeability of at least 100 $g/m^2 \cdot 24h$, more preferably at least 300 $g/m^2 \cdot 24h$, and most preferably at least 500 $g/m^2 \cdot 24h$.

Suitable support layer for use herein include two dimensional, planar micro and macro-porous films; macroscopically expanded films; formed apertured films; nonwoven and woven layers. According to the present invention the apertures in said layer may be of any configuration, but are preferably spherical or oblong and may also be of varying dimensions. The apertures preferably are evenly distributed across the entire surface of the layer, however layers having only certain regions of the surface having apertures are also envisioned.

Suitable two dimensional porous planar layers may be made of any material known in the art, but are preferably manufactured from commonly available polymeric materials. Suitable materials are XMP-1001 of Minnesota Mining and Manufacturing Company, St. Paul, Minnesota, USA and Exxaire XBF-101W, supplied by the Exxon Chemical Company. As used herein the term two dimensional planar layer refers to layers having a depth of less than 1 mm, preferably less than 0.5 mm, wherein the apertures have an average uniform diameter along their length and which do not protrude out of the plane of the layer. The apertured materials for use as the support layer may be produced using any of the methods known in the art such as described in EPO 293 482 and the references therein. In addition the dimensions of the apertures produced by this method may be increased by applying a force across the plane of the layer (i.e. stretching the layer).

Suitable apertured formed films include films which have discrete apertures which extend beyond the horizontal plane of the garment facing surface of the layer towards the core thereby forming protuberances. The protuberances have an orifice located at its terminating end. Preferably said protuberances are of a funnel shape, similar to those described in US 3,929,135. The apertures located within the plane and the orifices located at the terminating end of protuberance themselves maybe circular or non circular provided the cross sectional dimension or area of the orifice at the termination of the protuberance is smaller than the cross sectional dimension or area of the aperture located within the garment facing surface of the layer. Preferably said apertured performed films are uni directional such that they have at least substantially, if not complete one directional fluid transport towards the core.

Suitable macroscopically expanded films for use herein include films as described in for example in US 4,637,819 and US 4,591,523.

Preferred support layers for use herein include woven and nonwoven layers.

The composites of this preferred embodiment according to the present invention are particularly advantageous as they allow the possibility of providing a composite wherein the thermoplastic composition may be coated onto the support layer/substrate as a layer with the desired thickness. Typical coating conditions and apparatuses known in the art for the direct coating of low viscosities hot melts can be readily utilised in order to provide the thermoplastic composition at the desired thickness.

A possible method for forming a composite laminate by coating the thermoplastic composition onto a substrate acting as a support layer is described in PCT application WO 96/25902.

At least at the coating temperature, the thermoplastic composition in form of a layer preferably exhibits adhesive properties on the supportive substrate in order to form the preferred composite such that no additional adhesive is required to achieve a permanent attachment between the thermoplastic composition and the substrate.

Preferably the moisture vapour permeable, liquid impervious composite structures, have an overall moisture vapour transfer rate of at least 100 $g/m^2 \cdot 24h$, more preferably at least 300 $g/m^2 \cdot 24h$, and most preferably at least 500 $g/m^2 \cdot 24h$.

The support layer as defined herein has a first and a second opposing surface. The first surface of the support layer is those facing the thermoplastic film of the composite structure and the second surface of the support layer is those opposite said first surface. According to the present invention the support layer of the composite structure is provided on the surface called herein second surface of the support layer with non-slipping properties.

The term 'non-slip' is used generally herein to describe liquid impermeable, moisture vapour permeable composite structures of the present invention, having a surface (typically the surface of the support layer not facing the thermoplastic film) which has been modified/treated so as to result in substantially reduced slippage when used in contact against human skin or another layer of an absorbent article or any other surfaces. The static coefficient of friction of this surface is generally more than 1, preferably more than 1.3, even more preferably more than 1.5 and most preferably more than 1.7.

The coefficient of friction of the non-slip surface of the composite structure of the present invention can be evaluated according to ASTM D 1894, Standard test Method for static and kinetic coefficients of friction of plastic film and sheeting, using a tensile tester, Instron model 6021 with the 6000 series control console with the "Peel, Tear, Block and Friction test" Software Program code 6100-014 (for selecting the test friction function). Test parameters are: Initial length 2mm, end point 100mm, test speed 150mm/min. The test is performed using the moving sled-stationary metallic plane (ASTM method of assembling C). Metallic plane (also called 'plate') having a dimension of 515*152*9 mm.

According to the present invention all measurements were made using a 6.25 cm by 6.25 cm square sled of 200g weight. The coefficient of friction used according to the present invention are the result of friction measurements made according to this ASTM test method between the support layer according to the present invention and a metallic plane covered by a reference material that is white 100% cotton weave style 429-w cut to 76mm*460mm supplied by testex 53947 Nettersheim Germany. For each test the cotton will be replaced with a new one. The cotton is clamped onto the metallic plane only on one end opposite to the traction (pull) of the sled.

The non-slip materials suitable for used according to the present invention may be any material including any latex or hot melt, coating that has sufficient skid-resistance properties to be able to match the coefficient of friction of the support layer according to the present invention when applied thereto. Alternatively the non-slip materials as described herein might be applied on the thermoplastic film too to increase the coefficient of friction of such material if desired.

The non-slip materials suitable to use herein include, but are not limited to, materials of the following groups of materials:

- Ethylene vinyl acetate copolymers that can be applied as a hot melt or as a water based coating. Preferred materials have at least 28% vinyl acetate,
- Polyvinyl acetate such as those typically used in water based emulsions
- Styrene-butadiene - applied in an emulsion or as a hot melt,
- Cellulose acetate butyrate - normally hot melt coatings,
- Ethyl cellulose - normally blended with a plasticizer and a resin and applied as hot melt,
- Acrylics - normally emulsion system that are not blended,
- Elastomers,
- Synthetic rubber hot melt - (Kraton® block copolymers having elastomeric and styrenic blocks, rubber, resin, plasticizer blends,
- other hot melts -polyethylenes (alone or blended), polyamides.

Preferred non-slip materials for use herein are ethylene-vinyl acetate copolymers, acrylic terpolymers of methacrylic acids, acrylic copolymers, ethylene-vinyl acetate/resin latex emulsions, ethylene-vinyl acetate hot-melt adhesives, synthetic rubber (block copolymers with elastomeric and styrenic components) hot melt adhesives and polyvinyl acetate/resin emulsions. Such materials are available from H.B. Fuller Company, E.1. Dupont and Findley Adhesives, among others.

In a preferred embodiment the non-slip materials for use herein are those delivering some water vapour permeability. Indeed such non-slip materials particularly suitable for use herein have a moisture vapour transfer rate of at least 100 g/m$^2$·24h, more preferably at least 300 g/m$^2$·24h, and most preferably at least 500 g/m$^2$·24h, when used in the form of continuous layer of a thickness of at least 0.5 μm.

Surprisingly by using such coating materials the moisture vapour permeability of the composite structures of the present invention is reduced only to a minimum extend or even not reduced at all. In other words, non-slip properties might be provided without impairing on the breathability properties of the composite structures. The preferred non-slip materials for use herein are hydrophilic non-slip materials per se or applied in hydrophilic form (e.g., water-based solution/dispersion or emulsion) which have the ability to transfer substantial amounts of moisture vapour through their thickness by absorbing water on one side where the moisture vapour concentration is higher, and desorbing or evaporating it on the opposite side of their thickness where the moisture vapour concentration is lower.

Particularly suitable non-slip moisture vapour permeable materials for use herein include moisture permeable polymeric resins such as an elastomer containing urethane bonds, which are permeable to passage of moisture vapour through their thickness. Such elastomer containing urethane bonds include silicones (e.g., heat-cured silicones, condensation-cured silicones and RTV silicones) polyurethanes. A preferred elastomer is RTV 863 from GE Silicones, Inc., Waterford, NY.

Other particularly suitable non-slip moisture vapour permeable materials include copolymers of alkyl ester of acrylic acid with comonomers. Such alkyl ester of acrylic acid copolymer based materials are particularly suitable has they combine the benefits of easy processability, delivering soft hand or feel, while being odour free and colour free (in contrast to for example elastomeric polymers which usually have a non desirable odour and impair a yellow/brunish colour to the support layer treated therewith which may not be acceptable by the user as not contributing to the desire of discretion of the product). Advantageously such alkyl ester of acrylic acid copolymer based materials confer non-slip properties not only to the composite structures of the present invention in dry conditions but also in wet conditions (typically upon accidental wet/soil through occurrence). Also such copolymer based materials have the ability to cure at room temperature. In other words crosslinking of the copolymer based material will not be affected during normal usage at room temperature, thereby contributing to increased non-slip durability in normal usage conditions. Additionally such copolymer based materials are non tacky at room temperatures, thereby providing excellent non-slip properties without stickiness. Indeed such polymer based materials have a glass transition temperature (Tg) which is below -40 degree Celsius. Below such a temperature the polymer based materials are stiff and become softer above. At usage temperatures (room temperatures) they have enhanced softness.

Such copolymer based materials might contain from 55% to 90% by weight of an alkyl ester of acrylic acid, wherein the alkyl group may contain from 1 to 8 carbon atoms, from 5% to 40% by weight of acrylonitrile and from 0.5% to 10% by weight of a crosslinking comonomer, the total of course being 100%. Typical crosslinking comonomer for use herein include N-methylol acrylamide, N-methylol methacrylamide, glycidyl acrylate, glycidyl methacrylate and the like.

Such copolymer might be prepared by any suitable polymerization method. Usually the polymerization is carried out in an organic solvent medium by heating the mixture of comonomers at the reflux temperature of the solvent, thus insuring a constant temperature and dissipation of the heat of reaction in evaporating the solvent. Solvent soluble free radical initiating catalysts of the peroxide or azo type are utilised in these polymerisation. If the resulting polymer is insoluble in the solvent, it can be filtered, washed and thereafter dissolved in a solvent in which it is soluble. The copolymers can be utilised in the form of the lacquers wherein they were prepared, or these lacquers, in turn, may be converted to aqueous emulsions prior to their application the support layer. Thus, lacquers of these copolymers may be readily emulsified by adding an aqueous solution of an emulsifier, such as polyvinyl alcohol, morpholine-oleic acid mixtures and the like, to the lacquers while the lacquers is being vigorously agitated. The organic solvent is then removed from the mixture by flash distillation or any other suitable method. The polymerization may also be carried out by dispersing the comonomers as fine droplets in a large volume of water through the use of emulsifying agents like surfactants. Then the polymerization is effected in the presence of water soluble catalysts, such as, for example, the persulfates. At any rate the solid contain of the copolymer formulae may range from 30% to 65% by weight.

In addition to the copolymers described previously, the non-slip copolymer based material may also include such other ingredients as inert fillers, pigments, catalytic agents, antioxidants, ultra violet stabilisers, diluents, thermosetting resins, plasticizers and the like if desired.

Example of commercially available alkyl ester of acrylic acid based copolymer is Nacrylic X4445® from National Starch. Such copolymers are fully described in US 3 336 149, which is incorporated herein by reference.

The non-slip material might be applied by any conventional coating method known to those skilled in the art including spraying, screen or flock printing or by rod coating or gravure printing processes.

Typically the non-slip material like alkyl ester of acrylic acid based copolymer commercially available from National Starch under the trade name Nacrylic X4445®, in a dispersed hydrophilic emulsion form (the polymer is a surfactant stabilized polymer-containing water based emulsion) is applied as a surface coating to the surface of the support layer, preferably nonwoven using airless spray technology, which utilises hydraulic pressure instead of air to atomise and spray. This can be undertaken with the dispersion at ambient temperature or by pre-heating up to 90 degrees centigrade. The dispersion can be sprayed at a non volatile content as supplied from the manufacturer (typically 35 - 60 %) or upon dilution with water to achieve the required dry binder add on to the support layer like nonwoven. Drying and curing of the polymer dispersion is achieved by the application of heat, typically by passing the bonded web through an Industrial scale oven until the desired end use characteristics have been attained.

The non-slip coating materials might be applied onto the surface of the support layer in any conventional way, being continuous or discontinuous. By 'discontinuous' application it is meant herein that portions of the support layer surface are left uncoated by the non-slip material. This uncoated portions of the support layer therefore may contribute to maintain the high moisture vapour permeability of the composite structures of the present invention. In the embodiments herein wherein the non-slip materials used has some moisture vapour permeable properties per se the application pattern chosen (discontinuous or continuous) only slightly influence the moisture vapour permeability of the composite structure.

The non-slip coating materials might be applied in the forms of small dots or domes (in circular forms, polygonal forms, square, pentagonal and the like) at spaced intervals or may be intersecting lines in various patterns such a grid pattern, straight rows, oblique rows or any other arrangements according to the intended application. The non-slip material may be applied in a discontinuous pattern extending across only a portion of the surface area of the total surface of the support layer, preferably having a size and distribution appropriate to cover between at least 1% and 98% of the total surface area of the support layer to which it is applied to. More preferably the non-slip material covers about 3% to 85% of the total surface area of the support layer and more preferably between 10% and 70%.

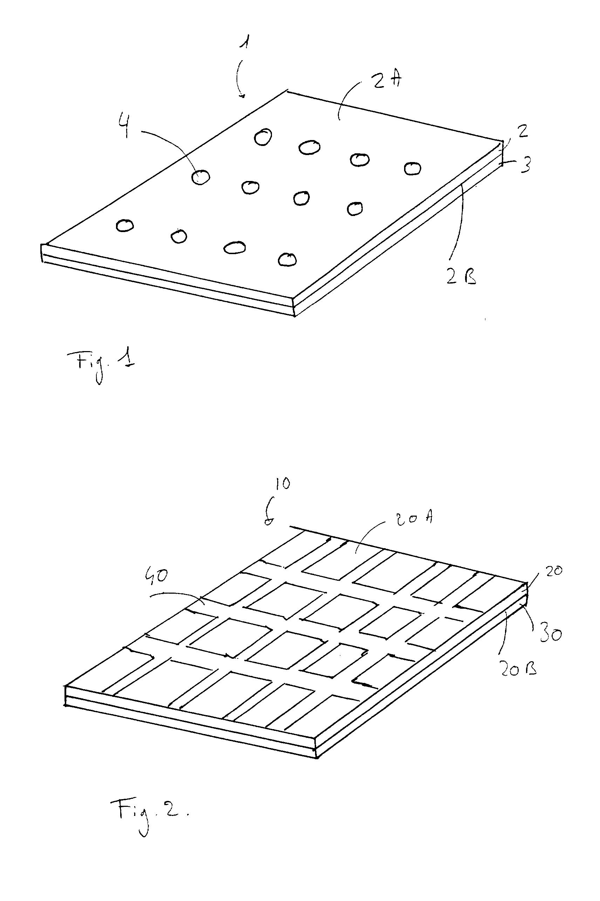

As can be seen from Figures 1 and 2, the support layer 2 or 20 has a first and a second opposing surfaces 2B and 2A or 20B and 20A, wherein the first surface 2B or 20B of the support layer 2 or 20 is facing the hydrophilic thermoplastic film 3 or 30 and the second surface 2A or 20A comprises the non-slip material in a discontinuous pattern, which pattern comprises a plurality of small dot-like or dome-like projections 4 or is a grid-like pattern 40.

In one embodiment herein wherein a discontinuous pattern of non-slip material is applied onto the composite structure of the present invention, the pattern selected for the application of the non-slip material may intentionally be directionally asymmetric in order to provide for different coefficients of friction in different directions. This condition may be particularly desirable for certain applications which might benefit from increased resistance to slippage in one direction while allowing a greater amount of slippage in another direction.

The composite structures of the present invention are particularly suitable to be used as the backsheet of disposable absorbent articles especially sanitary napkins, breast pads, perspiration pads and panty liners.

The term "disposable" is used herein to describe articles which are not intended to be laundered or otherwise restored or reused as an absorbent article (i.e., they are intended to be discarded after a single use and, preferably to be recycled, composted or otherwise disposed of in an environmentally compatible manner).

Such disposable absorbent articles will typically comprise components known to the skilled person such as a liquid pervious topsheet, an absorbent core and backsheet. The non-slip surface of the composite structure when used as backsheet of an absorbent article like a breast pad is typically facing the garment of the wearer of the article, e.g., the bra in case of breast pads. If desired it might also be used in combination with a conventional garment attachment adhesive. Such adhesive attachment means might be desirable for keeping the breast pad attached onto the bra during some operation (like opening partially the bra or opening the cover of the bra for inspection by the use of the pad during use).

The use of such a composite structure according to the present invention as the backsheet of absorbent articles like breast pads has the advantage to participate to the thinness and outstanding moisture vapour permeability properties and protection properties of the overall article (leakage prevention), while being also softer and more comfortable for the wearer and providing stay in place properties upon usual usage conditions.

Such composite structures may also be used in other disposable applications like socks, gloves, surgical fabrics or medical protective clothing such as surgical drapes, surgical gowns and containment gowns. Still a particular application is a disposable moisture vapour permeable liquid impermeable mattress cover which comprises a composite structure as described herein comprising the thermoplastic film, in use, to directly contact a mattress such that the outer surface of the support layer (also called herein second surface of the support layer) being treated with the non-slip material is facing the user's skin. The non-slip surface will inhibit an ill person from inadvertently slipping from a chair or bed as a result of perspiration or incontinence rendering the mattress surface excessively slippery. The term mattress, as used herein refers to a fabric case filled with resilient material, such as for example cotton, hair, feathers, foam rubber or an arrangement of coil springs, and therefore comprises particularly bed mattresses, but also pillows, cushions, comforters, duvets, upholstered portions of beds (such as headboards) or of sofas or armchairs. Such disposable mattress covers can be comprised solely of the composite structure of the present invention it if however preferred that such composite structures are used in combination with one or more other materials to create a layered composite structure comprised in the mattress cover. The mattress cover can include two or more components of a same specific thermoplastic composition as described herein or different specific thermoplastic composition as described herein.

Moisture Vapour Permeability Test

The Vapour permeability test is utilised to quantify the vapour transmission properties of breathable film/layer or composite structure according to the present invention

Basic Principle of the Methods

The basic principle of the test is to quantify the extent of water vapour transmission of the layer/film or composite structure to be tested. The test method that is applied is a standard one, namely ASTM E 96 - 80, Procedure B - Water Method at 23°C. The test is performed in a stable temperature/humidity laboratory maintained at a temperature of 23° C at 50% RH for a period of 24 hours.

The vapour permeability value is determined as the weight loss divided by the open area of the sample holder and quoted per day.

i.e. Vapour Permeability = Weight Loss (g) / (m$^2$/ 24 hrs)

Example 1

An example of thermoplastic film of a composite structure according to the present invention is obtained by compounded a polyether-amide block copolymer available from Elf Atochem (France) under the trade name Pebax 2533® with acetyltributyl citrate available from Reilly Chemicals under trade name Citroflex A4®, resins available from Hercules Inc. under code name Res A-2690 and Res A-2691 and Irganox 1010® and PS800® (antioxidant agent) available from Ciba-Geigy.

The thermoplastic film composition in percent by weight is as follows:

Resin available from Hercules Inc. under code name Res A-2690 (14.85%)

Resin available from Hercules Inc. under code name Res A-2691 (14.85%)

These resins are described in our European application No. 00116284.1

Acetyltributyl citrate Citroflex A4® from Reilly Chemicals (25%)

Polyether Block Amides Pebax 2533® from Elf Atochem (45%)

Irganox 1010® (0.15%) from Ciba Geigy

Irganox PS 800® (0.15%) from Ciba Geigy

The blend was melt coated at 175°C to obtain a film having a thickness of about 50µm. The film was then laminated directly onto a support layer constituted by nonwoven thermalbonded 100% Polypropylene supplied by liesstoffwerk Chr. H. Sandier Gmbh& Co.KG under material code: Sawabond 4124®.

The complex viscosity η* is measured using a Rheometer RDA-II available from Rheometrics Co. Moisture vapour permeability is measured as Water Vapour Transmission Rate (WVTR) at 23°C according to the ASTM E-96 "Upright Cup" method. The softening point of the resins is measured according to the Ring and Ball ASTM E28 method.

Then the nonwoven support layer of the composite structure is provided with non-slip properties by being treated with self-crosslinking acrylic copolymer emulsion commercially available from National Starch under material code: Nacrylic X4445®.

Indeed Nacrylic X4445® (commercially available at a solid content of 45%) is diluted with water (90g of polymer at 45% of solid content with 10g of water). This resulting solution is sprayed onto the nonwoven already coated to the thermoplastic film. The spray operation is conducted at room temperature (23°C +-2°C) by spraying 21.73g/m2 (wet polymer) which corresponds to 8.8g/m2 (dry polymer) after drying at room temperature.

This composite structure might be used as a backsheet of absorbent articles like breast pads. In such articles the support layer with the non-slip material is intended to face, in use, the garment/bra, and to directly contact it.

**Claims**

1.  A non-slip, moisture vapour permeable, liquid impervious composite structure comprising a hydrophilic thermoplastic film and a support layer, the support layer having a first and a second opposing surfaces, wherein the first surface of the support layer is facing the hydrophilic thermoplastic film and the second surface has a coefficient of friction greater than 1.

2.  A non-slip composite structure according to claim 1, wherein the second surface of the support layer has a coefficient of friction greater than 1.3, more preferably greater than 1.5, and most preferably more than 1.7.

3.  A non slip composite structure according to any of the preceding claims wherein the second surface of the support layer comprises a non-slip material applied thereto, said material being a non-slip latex or hot melt coating typically selected from the group consisting of ethylene vinyl acetate copolymers, polyvinyl acetate, styrene-butadiene, cellulose acetate butyrate, ethyl cellulose, acrylic acid copolymers, elastomers, synthetic rubber hot melt, hot melt polyethylenes, hot melt polyamides and mixtures thereof.

**4.** A non-slip composite structure according to any of the preceding claims wherein the second surface of the support layer comprises a moisture vapour permeable non-slip material applied thereto, preferably an elastomer containing urethane bonds and/or an alkyl ester of acrylic acid copolymer based material.

**5.** A non slip composite structure according to any of the preceding claims wherein the second surface of the support layer comprises a non-slip material applied thereto in a discontinuous way.

**6.** A non-slip composite structure according to any of the preceding claims, wherein said thermoplastic film comprises from 5% to 100% by weight of a polymer or mixture of polymers.

**7.** A non-slip composite structure according to claim 6, wherein said polymer is selected from the group consisting of polyurethanes, poly-ether-amides block copolymers, polyethylene-acrylic acid copolymers, polyethylene oxide and its copolymers, ethylene acrylic esters copolymers, poly lactide and copolymers, polyamides, polyester block copolymers, sulfonated polyesters, poly-ether-ester block copolymers, poly-ether-ester-amide block copolymers, polyacrylates, polyacrylic acids and derivatives, ionomers, polyethylene-vinyl acetate with a vinyl acetate content of more than 28 weight %, polyvinyl alcohol and its copolymers, polyvinyl ethers and their copolymers, poly-2-ethyl-oxazoline and derivatives, polyvinyl pyrrolidone and its copolymers, thermoplastic cellulose derivatives, and mixtures thereof.

**8.** A composite structure according to any of the preceding claims, wherein said thermoplastic film further comprises from 0% to 95%, preferably from 20% to 90% by weight of a plasticiser or mixtures thereof.

**9.** A composite structure according to claim 8, wherein said plasticiser is selected from the group consisting of citric acid esters, tartaric acid esters, glycerol and its esters, adipates, sebacates, sorbitol, epoxidized vegetal oils, polymerised vegetal oils, polyols, phthalates, liquid polyesters, glycolates, p-toluene sulfonamide and derivatives, glycols and polyglycols, sorbitan esters, phosphates, monocarboxylic fatty acids ($C_8$-$C_{22}$) and their derivatives, and mixtures thereof.

**10.** A composite structure according to any of the preceding claims, wherein said thermoplastic film further comprises from 0% to 60%, preferably from 2% to 60%, more preferably from 5% to 50%, and most preferably from 10% to 40%, by weight of a tackifier resin or blend of tackifier resins.

**11.** A composite structure according to any of the preceding claims, which has a water vapour transfer rate of at least 100g/(m$^2$.24hrs.).

**12.** A composite structure according to any of the preceding claims, wherein said support layer is selected from wovens, nonwovens and apertured films.

**13.** An absorbent article comprising a composite structure according to any one of the preceding claims.

**14.** An absorbent article according to claim 13, wherein the composite structure is used as the backsheet of the absorbent article, the second surface of the support layer directly facing the garment of the wearer of such an article.

**15.** An absorbent article according to claims 13 or 14 wherein said article is a pantiliner, sanitary napkin, diaper, perspiration pad, incontinence product, protective bedding covers, protective clothing, a mattress cover or a breast pad.

Fig. 1

Fig. 2.

## EUROPEAN SEARCH REPORT

**European Patent Office**

Application Number

EP 00 12 2216

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | US 5 948 707 A (SCHMIEDER MICHAEL A ET AL) 7 September 1999 (1999-09-07) | 1-5,7, 11-15 | B32B27/08 B32B27/12 |
| Y | * column 4, line 41; claims 1-5,8,14-17,24-28 * | 6,8-10 | D06N7/00 A61L15/42 A61L15/22 |
| D,Y | EP 0 963 760 A (PROCTER & GAMBLE) 15 December 1999 (1999-12-15) | 1,3,6-15 | |
| X | * claims 1,3,4,6-10 * | 1 | |
| Y | US 4 667 661 A (SCHOLZ MATTHEW T ET AL) 26 May 1987 (1987-05-26) * column 2, line 52; claims 1,8,9,19,21 * | 1,3,6,7, 12-15 | |
| Y | US 5 840 812 A (SCHULTZE DIRK) 24 November 1998 (1998-11-24) * the whole document * | 6-8,11 | |
| Y | US 5 950 264 A (WYNER DANIEL M ET AL) 14 September 1999 (1999-09-14) * claims 1,7,8 * | 6-8, 12-15 | |
| A | EP 1 040 799 A (PROCTER & GAMBLE) 4 October 2000 (2000-10-04) * claims 1,2,7-10 * | 11-15 | TECHNICAL FIELDS SEARCHED (Int.Cl.7) B32B D06N A61L |
| A | DATABASE WPI Section Ch, Week 198408 Derwent Publications Ltd., London, GB; Class A26, AN 1984-045193 XP002164274 & JP 59 005058 A (SHINETSU CHEM IND CO LTD), 11 January 1984 (1984-01-11) * abstract * | 1 | |

The present search report has been drawn up for all claims

| Place of search | Date of completion of the search | Examiner |
|---|---|---|
| THE HAGUE | 29 March 2001 | Derz, T |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 00 12 2216

This annex lists the patent family members relating to the patent documents cited in the above-mentioned European search report.
The members are as contained in the European Patent Office EDP file on
The European Patent Office is in no way liable for these particulars which are merely given for the purpose of information.

29-03-2001

| Patent document cited in search report | | Publication date | Patent family member(s) | | Publication date |
|---|---|---|---|---|---|
| US 5948707 | A | 07-09-1999 | AU | 2997599 A | 27-09-1999 |
| | | | EP | 1062094 A | 27-12-2000 |
| | | | WO | 9946118 A | 16-09-1999 |
| EP 0963760 | A | 15-12-1999 | AU | 3842599 A | 30-12-1999 |
| | | | AU | 4338299 A | 30-12-1999 |
| | | | BR | 9911122 A | 20-02-2001 |
| | | | EP | 1085911 A | 28-03-2001 |
| | | | WO | 9964077 A | 16-12-1999 |
| | | | WO | 9964078 A | 16-12-1999 |
| US 4667661 | A | 26-05-1987 | AT | 86123 T | 15-03-1993 |
| | | | AT | 122898 T | 15-06-1995 |
| | | | AU | 591707 B | 14-12-1989 |
| | | | AU | 6291586 A | 09-04-1987 |
| | | | BR | 8604689 A | 23-06-1987 |
| | | | CA | 1293656 A | 31-12-1991 |
| | | | CN | 86106623 A,B | 13-05-1987 |
| | | | DE | 3650329 D | 29-06-1995 |
| | | | DE | 3650329 T | 28-09-1995 |
| | | | DE | 3687879 A | 08-04-1993 |
| | | | DE | 3687879 T | 17-06-1993 |
| | | | DE | 221669 T | 07-04-1988 |
| | | | DK | 458486 A | 05-04-1987 |
| | | | EP | 0221669 A | 13-05-1987 |
| | | | EP | 0494083 A | 08-07-1992 |
| | | | ES | 2002794 A | 01-10-1988 |
| | | | HK | 71794 A | 29-07-1994 |
| | | | JP | 2026823 C | 26-02-1996 |
| | | | JP | 5065193 B | 17-09-1993 |
| | | | JP | 62087162 A | 21-04-1987 |
| | | | KR | 9003365 B | 16-05-1990 |
| | | | NO | 863632 A,B, | 06-04-1987 |
| | | | NZ | 217549 A | 27-10-1989 |
| | | | TR | 22932 A | 29-11-1988 |
| | | | US | 4774937 A | 04-10-1988 |
| | | | ZA | 8606779 A | 27-04-1988 |
| US 5840812 | A | 24-11-1998 | DE | 4442380 A | 30-05-1996 |
| | | | CA | 2163722 A | 30-05-1996 |
| | | | EP | 0714950 A | 05-06-1996 |
| US 5950264 | A | 14-09-1999 | AU | 2094595 A | 18-09-1995 |
| | | | CA | 2184625 A | 08-09-1995 |
| | | | EP | 0748178 A | 18-12-1996 |
| | | | JP | 9509859 T | 07-10-1997 |

For more details about this annex : see Official Journal of the European Patent Office, No. 12/82

**ANNEX TO THE EUROPEAN SEARCH REPORT
ON EUROPEAN PATENT APPLICATION NO.**                EP 00 12 2216

This annex lists the patent family members relating to the patent documents cited in the above-mentioned European search report.
The members are as contained in the European Patent Office EDP file on
The European Patent Office is in no way liable for these particulars which are merely given for the purpose of information.

29-03-2001

| Patent document cited in search report | Publication date | Patent family member(s) | Publication date |
|---|---|---|---|
| US 5950264 A | | WO 9523541 A | 08-09-1995 |
| EP 1040799 A | 04-10-2000 | AU 4191000 A<br>WO 0059432 A | 23-10-2000<br>12-10-2000 |
| JP 59005058 A | 11-01-1984 | JP 62001823 B | 16-01-1987 |

EPO FORM P0459

For more details about this annex : see Official Journal of the European Patent Office, No. 12/82